# EUROPÄISCHE PATENTANMELDUNG

(11) **EP 1 086 652 A1**
(43) Veröffentlichungstag der Anmeldung: **28.03.2001**
(21) Anmeldenummer: 00203243.1
(22) Anmeldetag: 18.09.2000
(51) Int. Cl.: A61B 6/00

(54) **Verfahren und Vorrichtung zur Ermittlung eines dreidimensionalen Bilddatensatzes eines sich periodisch bewegenden Körperorgans**

(30) Priorität: 25.09.1999 DE 19946092
(71) Anmelder: Philips Corporate Intellectual Property GmbH, 52064 Aachen (DE); Koninklijke Philips Electronics N.V., 5621 BA Eindhoven (NL)
(72) Erfinder: Rasche, Volker, Philips Corp. Intel. Prop. GmbH, 52064 Aachen (DE); Grass, Michael, Philips Corp. Intel. Prop. GmbH, 52064 Aachen (DE)
(74) Vertreter: Volmer, Georg, Dipl.-Ing.

(57) **Zusammenfassung**

Die Erfindung betrifft ein Verfahren und eine Vorrichtung zur Ermittlung eines dreidimensionalen Bilddatensatzes eines sich periodisch bewegenden Körperorgans (11) eines Patienten (5) mittels einer eine Röntgenquelle und einen Röntgendetektor (3) aufweisenden Röntgeneinrichtung (1), bei dem gleichzeitig zur Ermittlung der Projektionsdatensätze (D₀, D₁, ..., D₁₆) ein im Zusammenhang mit der periodischen Bewegung des Körperorgans (11) stehendes Bewegungssignal (H,B) ermittel wird. Zur Verbesserung eines solchen Verfahrens bzw. einer solchen Vorrichtung, insbesondere zur Verbesserung des Aufbaus und zur Verkürzung der für die Datenverarbeitung erforderlichen Zeit bei möglichst niedriger Strahlenbelastung für den Patienten und bei möglichst hoher Bildqualität, wird erfindungsgemäß vorgeschlagen, dass nacheinander die für die Ermittlung des dreidimensionalen Bilddatensatzes erforderlichen Projektionsdatensätze (D_{0,}D₁,...., D₁₆) aus unterschiedlichen in einer Ebene liegenden Röntgenpositionen (p₀, p₁,...., p₁₆) ermittelt werden, dass die Röntgeneinrichtung mittels des Bewegungssignals (H,B) derart gesteuert wird, dass in jeder für die Ermittlung des dreidimensionalen Bilddatensatzes erforderlichen Röntgenposition (p₀, p₁,....., p₁₆) ein Projektionsdatensatz (D₀, D₁,...., D₁₆) während einer bewegungsarmen Phase des Körperorgans (11) ermittelt wird, und dass die während der bewegungsarmen Phase ermittelten Projektionsdatensätze (D0, D₁,...., D₁₆) zur Ermittlung des dreidimensionalen Bilddatensatzes verwendet werden.

## Beschreibung

Die Erfindung betrifft ein Verfahren zur Ermittlung eines dreidimensionalen Bilddatensatzes eines sich periodisch bewegenden Körperorgans eines Patienten gemäß dem Oberbegriff des Anspruchs 1 sowie eine Röntgeneinrichtung, insbesondere zur Durchführung eines solchen Verfahrens, gemäß dem Oberbegriff des Anspruchs 12.

Zur Ermittlung eines dreidimensionalen Bilddatensatzes eines Objektes mittels einer Röntgeneinrichtung ist es erforderlich, mehrere Projektionsdatensätze aus unterschiedlichen Röntgenpositionen zu ermitteln und daraus mittels eines geeigneten Rekonstruktions-Algorithmus den dreidimensionalen Bilddatensatz zu berechnen, aus dem sich ein gewünschtes Bild des Objekts erstellen läßt. Die Qualität eines derartigen Bildes hängt dabei insbesondere auch davon ab, wie viele Projektionsdatensätze, d.h. aus wie vielen verschiedenen Röntgenpositionen, ermittelt werden, wie hoch der Kontrast zwischen dem darzustellenden Objekt und dessen Umgebung ist und wie viel sich das Objekt während der Ermittlung der Projektionsdatensätze bewegt. All diese Faktoren können zu störenden Artefakten im darzustellenden Bild führen.

Das Problem eines zu geringen Kontrastes kann beispielsweise mit Hilfe eines Kontrastmittels gelöst werden. Sollen z.B. Bilder der Herzkranzgefäße erstellt werden, kann in diese mittels eines Katheters ein Kontrastmittel eingespritzt werden, das für etwa 4 Sekunden in den Herzkranzgefäßen bleibt, so dass diese in während dieser Zeitspanne ermittelten Projektionsdatensätze einen hohen Kontrast gegenüber ihrer Umgebung aufweisen. Allerdings ist es derzeit nicht möglich, während dieser Zeitspanne alle für die Ermittlung eines dreidimensionalen Bilddatensatzes erforderlichen Projektionsdatensätze zu ermitteln, so dass das Kontrastmittel mehrfach eingeführt und Projektionsdatensätze in mehreren Röntgenzyklen nacheinander ermittelt werden müssen. Es stellt sich dabei allerdings das weitere Problem, dass das Herz ständig pulsiert, also eine Eigenbewegung durchführt, und zusätzlich aufgrund der Atembewegung des Patienten merklich hin und her bewegt wird.

Auch andere interessierende Körperorgane wie beispielsweise die Leber oder das Gehirn bewegen sich periodisch im Körper, insbesondere aufgrund der pulsierenden Bewegung des Herzens und der Atembewegung des Patienten. Dies führt dazu, dass die ermittelten Projektionsdatensätze Informationen über das untersuchte Körperorgan in unterschiedlichen Bewegungsphasen enthalten und dass ein aus derartigen Projektionsdatensätzen ermittelter dreidimensionaler Bilddatensatz Artefakte enthält.

Aus der US 5,383,231 ist eine Computertomographie-(CT)-Anordnung bekannt, bei der Projektionsdatensätze während einer schraubenförmigen Abtastbewegung der Röntgenquelle und des Röntgendetektors um den Patienten erfaßt werden. Gleichzeitig und unabhängig davon wird ein Elektrokardiogramm des Patienten geschrieben, dessen Daten dazu verwendet werden, bei der Erstellung der CT-Bilder und eines dreidimensionalen Bilddatensatzes aus den Projektionsdatensätzen, den Tischvorschub des Patientenrisches während der Erfassung der Projektionsdatensätze zu berücksichtigen und nur die während einer bestimmten Herzbewegungsphase ermittelten Projektionsdatensätze auszuwerten. Um dort zu einem dreidimensionalen Bilddatensatz zu gelangen, müssen jedoch zunächst aus den Projektionsdatensätzen CT-Bilddatensätze berechnet werden, aus denen dann durch Interpolations-Algorithmen ein dreidimensionaler Bilddatensatz erstellt werden kann. Außerdem ist eine direkte und unmittelbare Verknüpfung zwischen der Erfassung des Elektrokardiogramms und der Erfassung der Projektionsdatensätze mittels der Röntgeneinrichtung nicht vorgesehen, so dass eine Reihe von Projektionsdatensätzen, die zu einer falschen Herzbewegungsphase ermittelt wurden, nicht ausgewertet werden.

Der vorliegenden Erfindung liegt die Aufgabe zugrunde, das eingangs genannte Verfahren und die eingangs genannte Röntgeneinrichtung zu verbessern, insbesondere den Aufbau einer solchen Röntgeneinrichtung zu vereinfachen und die Datenverarbeitung in kürzerer Zeit zu ermöglichen bei möglichst niedriger Strahlenbelastung für den Patienten und bei möglichst hoher Bildqualität.

Diese Aufgabe wird erfindungsgemäß durch ein Verfahren nach Anspruch 1 und eine Röntgeneinrichtung nach Anspruch 12 gelöst.

Zwar ist es grundsätzlich möglich, die Anzahl der Röntgenpositionen und der zu erfassenden Projektionsdatensätze zu verringern und einzelne Projektionsdatensätze durch interpolierte Daten zu ersetzen. Dies führt jedoch immer zu einer Verschlechterung der Bildqualität, und eine derartige Verringerung der ermittelten Daten kann nur bis zu einer bestimmten unteren Grenze durchgeführt werden, unterhalb derer verschiedene Effekte bei der Datenverarbeitung, wie z.B. der Überlagerungseffekt aufgrund zu niedriger Abtastrate, die Erstellung eines dreidimensionalen Bilddatensatzes nicht oder kaum möglich machen. Erfindungsgemäß ist deshalb vorgesehen, in jeder Röntgenposition, die für die Ermittlung eines dreidimensionalen Bilddatensatzes erforderlich ist, einen Projektionsdatensatz des zu untersuchenden Körperorgans zu ermitteln, und zwar jeweils während einer bewegungsarmen Phase des sich periodisch bewegenden Körperorgans. Das sich periodisch bewegende Körperorgan kann gleichzeitig das zu untersuchende Körperorgan sein oder ein Körperorgan, das eine ebenfalls periodische Bewegung des zu untersuchenden Körperorgans bewirkt (z.B. kann das zu untersuchende Körperorgan das Gehirn sein, dessen periodische Bewegung im Zusammenhang steht mit der periodischen Bewegung des Herzens derart, dass das Gehirn eine Bewegung mit derselben Periode wie das Herz ausführt). Zur Steuerung der Röntgeneinrichtung und der Datenerfassung wird erfindungsgemäß das gleichzeitig ermittelte Bewegungssignal benutzt. Die Ermittlung der Projektionsdatensätze braucht dabei nicht in der Reihenfolge der durchlaufenen Röntgenpositionen erfolgen, sondern es können auch einzelne oder alle Röntgenpositionen mehrfach durchlaufen und dabei jeweils ein Projektionsdatensatz ermittelt werden, wobei nur sicherzustellen ist, dass in jeder Röntgenposition mindestens ein Projektionsdatensatz während einer bewegungsarmen Phase ermittelt wird.

Verschiedene vorteilhafte Ausgestaltungen des erfindungsgemäßen Verfahrens und der erfindungsgemäßen Röntgeneinrichtung sind in den weiteren Ansprüchen angegeben.

Besonders bevorzugt sind Ausgestaltungen, bei denen ein von der Atmung des Patienten abhängiges Atembewegungssignal und/oder ein von der Bewegung des Herzens abhängiges Herzbewegungssignal, insbesondere ein Elektrokardiogramm, ermittelt wird. Welches dieser Signale ermittelt und zur Steuerung der Röntgeneinrichtung und der Datenerfassung verwendet wird, hängt insbesondere davon ab, wodurch die Bewegung des zu untersuchenden Körperorgans beeinflußt wird. Wird beispielsweise das Herz betrachtet, so führt dies sowohl eine Rotations- als auch eine Translationsbewegung aus. Dabei ist die Bewegung stärker in der systolischen Phase, in der sich die Herzkammern zusammenziehen und das Blut aus dem Herzen herauspressen, als in der diastolischen Phase, in der sich die Kammern langsam wieder mit Blut füllen und das Herz sich ausdehnt. Überlagert ist diese Eigenbewegung des Herzens durch die Atembewegung, da das Herz beim Einatmen zum Kopf des Patienten hin verschoben und zum Rücken hin leicht verkippt wird, während es beim Ausatmen wieder in die ursprüngliche Lage zurückkehrt.

In weiteren Ausgestaltungen der Erfindung ist deshalb auch vorgesehen, ein von der Atembewegung des Patienten abhängiges Atembewegungssignal zu ermitteln und anhand dessen Projektionsdatensätze auszuwählen zur Ermittlung des dreidimensionalen Bilddatensatzes, die alle während der gleichen Atembewegungsphase, z.B. im ausgeatmeten Zustand, erfaßt wurden. Das Atembewegungssignal kann auch dazu benutzt werden, in unterschiedlichen Atembewegungsphasen ermittelte Projektionsdatensätze zur Ermittlung des dreidimensionalen Bilddatensatzes zu verarbeiten und dabei die Positionsverschiebungen des zu untersuchenden Körperorgans aufgrund der Atembewegung zu korrigieren. Dies setzt beispielsweise voraus, dass die Bewegung oder die Position des Körperorgans in jeder Atembewegungsphase bekannt ist oder modellhaft angenommen wird.

In einer anderen Ausgestaltung kann das Atembewegungssignal dazu benutzt werden, dem Patienten zu signalisieren, wenn eine gewünschte Atembewegungsphase, z.B. der ausgeatmete Zustand, erreicht ist, damit der Patient dann während dieser Atembewegungsphase möglichst lange Zeit die Luft anhält, so dass während dessen möglichst viele Projektionsdatensätze aus unterschiedlichen Röntgenpositionen ermittelt werden können.

Ein in Ausgestaltungen der Erfindung ermitteltes Herzbewegungssignal kann dazu verwendet werden, nur die während der diastolischen Phase der Herzbewegung, in der die Bewegung des Herzens deutlich geringer ist als in der systolischen Phase, akquirierten Projektionsdatensätze zur Ermittlung des dreidimensionalen Bilddatensatzes zu verwenden, oder überhaupt nur Projektionsdatensätze zu ermitteln, wenn sich das Herz gerade in der diastolischen Phase befindet. In der systolischen Phase ermittelte Projektionsdatensätze werden dann entweder nicht weiter verwendet oder sie werden gar nicht erfaßt, wozu die Röntgeneinrichtung entsprechend steuerbar ist.

Vorteilhafte Ausgestaltungen der Mittel zur Ermessung des Bewegungssignals sind in den Ansprüchen 13 bis 17 angegeben.

Die Erfindung wird nachfolgend anhand der Zeichnungen näher erläutert.
Es zeigen:
- Fig. 1: eine Röntgeneinrichtung gemäß der Erfindung,
- Fig. 2: eine Skizze verschiedener Röntgenpositionen während eines Röntgenzyklus,
- Fig. 3: ein Zeitdiagramm eines Herzbewegungssignals zur Verdeutlichung der Ermittlung der Projektionsdatensätze gemäß der Erfindung,
- Fig. 4: ein weiteres Zeitdiagramm eines Herzbewegungssignals zur Erläuterung einer weiteren Ausgestaltung des erfindungsgemäßen Verfahrens und
- Fig. 5: ein Zeitdiagramm eines Atembewegungssignals.

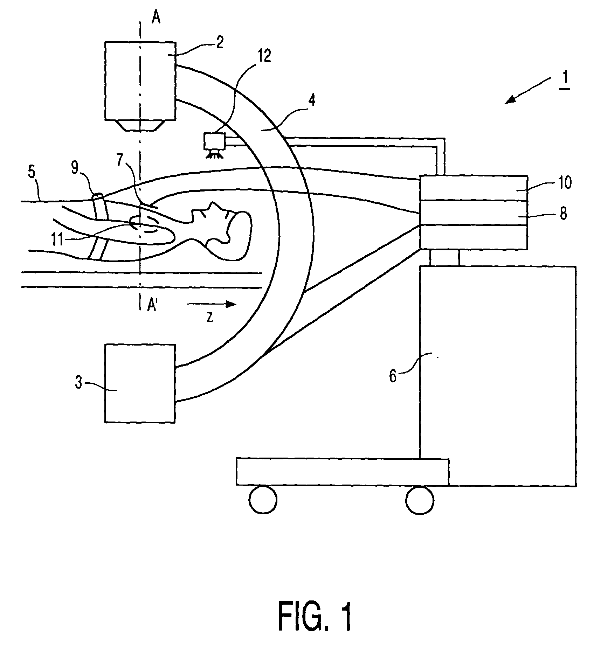

Die in Fig. 1 gezeigte Röntgeneinrichtung 1 weist eine Röntgenröhre 2 und einen zweidimensionalen Röntgendetektor 3, beispielsweise einen Bildverstärker, auf, die an einem C-Bogen 4 derart angeordnet sind, dass sie um einen Patienten 5 um die z-Achse rotierbar und um eine dazu senkrechte Achse verkippbar angeordnet sind. Die Steuerung der Röntgeneinrichtung 1 und die Verarbeitung der mit dem Röntgendetektor 3 erfaßten Daten erfolgt in der Steuer- und Recheneinheit 6. Zur Erfassung eines Elektrokardiogramms des Patienten 5 sind in dessen Brustbereich mit einer Elektrokardiographieeinrichtung 8 verbundene Elektroden 7 angeordnet. Die Atembewegung des Patienten 5 wird mittels eines durch die Atembewegung verformbaren Bauchgurtes 9 gemessen, die mit einer Atembewegungsmeßeinrichtung 10 zur Erstellung eines Atembewegungssignals verbunden ist. Das Elektrokardiogramm und das Atembewegungssignal werden von der Elektrokardiographieeinrichtung 8 bzw. der Atembewegungsmeßeinrichtung 10 online an die Steuer- und Recheneinheit 6 weitergeleitet, um unmittelbar bei der Steuerung der Röntgeneinrichtung 1 und der Datenerfassung Berücksichtigung zu finden.

Im gezeigten Fall befindet sich die Röntgeneinrichtung 1 in einer Röntgenposition für die Erfassung eines Projektionsdatensatzes des Herzens 11. Um alle Projektionsdatensätze, die für die Ermittlung eines dreidimensionalen Bilddatensatzes erforderlich sind, in der gleichen Atembewegungsphase ermitteln zu können, ist bei der gezeigten Ausführungsform eine Signaleinrichtung 12 vorgesehen, die dem Patienten 5 das Erreichen einer gewünschten Atembewegungsphase, z.B. des ausgeatmeten Zustandes, anzeigt, so dass dieser möglichst lange die Luft anhalten kann. Während dieser Zeit können dann Projektionsdatensätze des Herzens 11 aus unterschiedlichen Röntgenpositionen erfaßt werden, ohne dass der Eigenbewegung des Herzens 11 eine Bewegung aufgrund der Atmung des Patienten 5 überlagert ist.

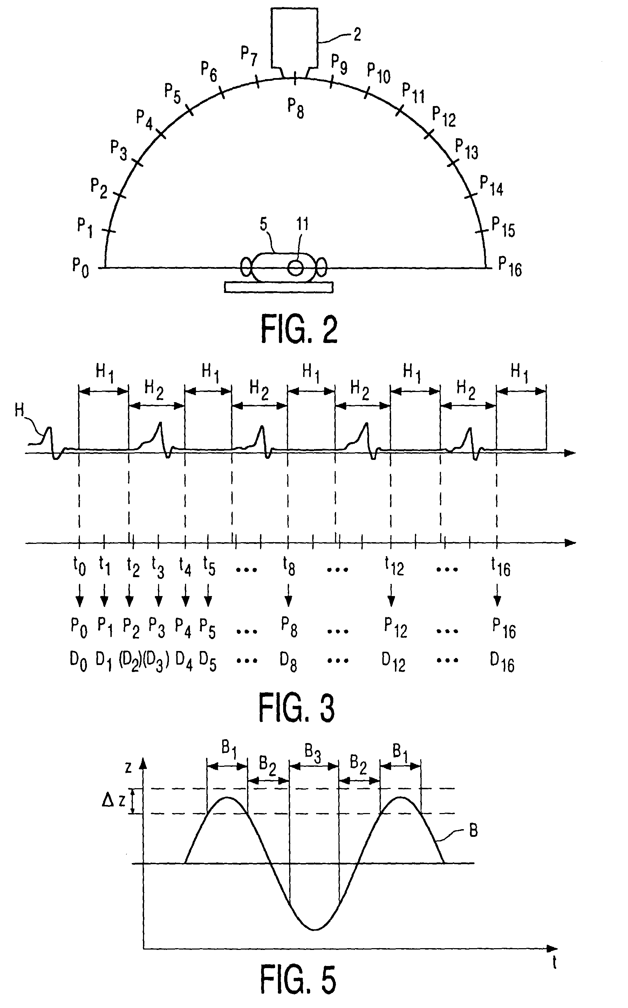

Eine Schnittdarstellung entlang der Linie A-A' zeigt Fig. 2. Dort sind symbolisch die verschiedenen auf einem Halbkreisbogen angeordneten Röntgenpositionen p₀ bis p₁₆ markiert, die die Röntgenröhre 2 nacheinander durchläuft und in denen jeweils ein Projektionsdatensatz erfaßt wird. Um einen dreidimensionalen Bilddatensatz zu erhalten, aus dem dann gewünschte Darstellungen des Herzens, z.B. einzelne Schichtbilder oder Bilder der Herzkranzgefäße erstellt werden können, ist es erforderlich, aus jeder der gezeigten Röntgenpositionen p₀ bis p₁₆ jeweils einen Projektionsdatensatz zu ermitteln, wobei die gezeigte Anzahl der Röntgenpositionen der Einfachheit halber gering gewählt ist.

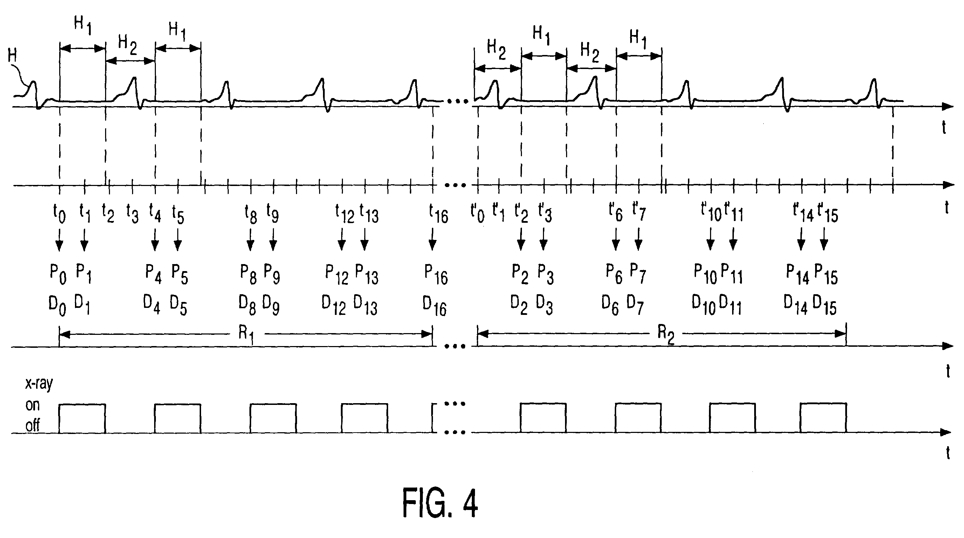

Um zu berücksichtigen, dass das Herz 11 während der Erfassung der Projektionsdatensätze eine Eigenbewegung durchführt, ist in einer Ausgestaltung der Erfindung vorgesehen, gleichzeitig zur Erfassung der Projektionsdatensätze als Bewegungssignal ein in Fig. 3 gezeigtes Elektrokardiogramm H zu messen. An einem unterhalb des Elektrokardiogramms H dargestellten Zeitstrahl sind die einzelnen Zeitpunkte t₀, t₁,...,t₁₆ markiert, zu denen sich die Röntgenröhre an den Röntgenpositionen p₀, p₁,...,p₁₆ befindet und zu denen jeweils ein Projektionsdatensatz D₀, D₁,...,D₁₆ ermittelt wird. Wie aus Fig. 3 unmittelbar zu ersehen ist, sind jeweils zwei Projektionsdatensätze (D₀,D₁; D₄, D₅;...) während der bewegungsarmen Phase H₁ (diastolische Phase) ermittelt worden, während jeweils zwei andere Projektionsdatensätze (D₂, D₃; D₆, D₇;...) während einer bewegungsreichen Phase H₂ (systolische Phase) ermittelt wurden. Um Bilder des Herzens von hoher Qualität und ohne Artefakte zu erhalten, ist es nicht möglich, die während der bewegungsreichen Phasen H₂ ermittelten Projektionsdatensätze (D₂,D₃, D₆, D₇,...) zur Ermittlung des dreidimensionalen Bilddatensatzes zu verwerten. Die verbleibenden verwendbaren Projektionsdatensätze (D₀, D₁, D₄, D₅,....) sind jedoch nicht ausreichend, um daraus einen dreidimensionalen Datensatz ausreichender Qualität zu rekonstruieren. Es werden deshalb alle Röntgenpositionen p₀ bis p₁₆ mehrfach nacheinander in jeweils einem Röntgenzyklus durchlaufen, wobei jeder Röntgenzyklus zu einem anderen Zeitpunkt innerhalb eines Herzzyklus beginnt, was anhand von Fig. 4 erläutert werden soll.

Dabei werden zunächst in einem ersten Röntgenzyklus R₁ (siehe dritten Zeitstrahl in Fig. 4) nacheinander die Röntgenpositionen p₀ bis p₁₆ durchlaufen, wobei nur die in den bewegungsarmen Phasen H₁ ermittelten Projektionsdatensätze D₀, D₁, D₄, D_{5,}...,D₁₆ verwendbar sind. Dieser erste Röntgenzyklus R₁ startet dabei zum Zeitpunkt t₀ zu Beginn einer bewegungsarmen Phase H₁. Nach einem Zeitintervall, um beispielsweise die Röntgeneinrichtung wieder in die Ausgangslage zu bringen, startet nun ein zweiter Röntgenzyklus R₂ zu einem Zeitpunkt t'₀, der etwa dem Beginn einer bewegungsreichen Phase H₂ entspricht. In der Röntgenposition p₂ zum Zeitpunkt t'₂ befindet sich die Röntgenröhre dann in einer bewegungsarmen Phase H₁, so dass der zu diesem Zeitpunkt ermittelte Projektionsdatensatz D₂, der im ersten Röntgenzyklus R₁ nicht ermittelt wurde oder - falls er ermittelt wurde - nicht verwendbar ist, zur Ermittlung des dreidimensionalen Bilddatensatzes verwendet werden kann. Gleiches gilt für die in diesem zweiten Röntgenzyklus R₂ ermittelten weiteren Projektionsdatensätze D₃, D₆, D₇, D₁₀,...., die dann alle während bewegungsarmer Phasen H₁ ermittelt werden. Durch diese Steuerung des Starts der einzelnen Röntgenzyklen wird erreicht, dass nach zwei Röntgenzyklen aus jeder Röntgenposition ein zu einer bewegungsarmen Phase H₁ ermittelter Projektionsdatensatz D₀ bis D₁₆ vorliegt, aus denen ein dreidimensionaler Bilddatensatz ermittelt werden kann.

Die Ermittlung der Projektionsdatensatze in den einzelnen Röntgenzyklen R₁, R₂ kann derart erfolgen, dass in jeder von der Röntgenquelle angefahrenen Röntgenposition p₀ bis p₁₆ jeweils ein Projektionsdatensatz D₀ bis D₁₆ ermittelt wird, dass aber nur die in bewegungsarmen Phasen H₁ ermittelten Projektionsdatensätze (D₀, D₁, D₄, D₅... im ersten Röntgenzyklus; D₂, D₃, D₆, D₇... im zweiten Röntgenzyklus) weiter verwendet werden. In einer alternativen Ausgestaltung kann jedoch auch vorgesehen sein, dass überhaupt nur in bewegungsarmen Phasen H₁ Projektionsdatensätze ermittelt werden, wobei jedoch alle Röntgenpositionen p₀ bis p₁₆ kontinuierlich durchlaufen werden. Dies bedeutet, dass im ersten Röntgenzyklus beispielsweise die Positionen p₂ und p₃ in einer bewegungsreichen Phase H₂ mit der Röntgenröhre zwar angefahren, dort aber kein Projektionsdatensatz erfaßt wird, beispielsweise indem die Röntgenröhre in diesen Positionen ausgeschaltet bleibt. Aus dem untersten Zeitstrahl in Fig. 4 ist ersichtlich, dass bei dieser Ausgestaltung der Erfindung die Röntgenröhre jeweils nur in bewegungsarmen Phasen H₁ eingeschaltet wird, während sie in bewegungsreichen Phasen H₂ ausgeschaltet bleibt. Die Steuerung dieses Ein-/Ausschaltens der Röntgenröhre erfolgt dabei mittels des Elektrokardiogramms H. Dies hat den Vorteil, dass der Patient während bewegungsreicher Phasen H₂ nicht unnötiger Weise mit Röntgenstrahlung belastet wird.

Bei der anhand von Fig. 4 erläuterten Ausgestaltung des erfindungsgemäßen Verfahrens können alle erforderlichen Projektionsdatensätze D₀ bis D₁₆ innerhalb von zwei Röntgenzyklen erfaßt werden. Dies muß jedoch nicht notwendig so sein, es können auch mehr Röntgenzyklen erforderlich sein, abhängig von dem Verhältnis der Zeitdauer der bewegungsarmen Phase H₁ zur Zeitdauer der bewegungsreichen Phase H₂. Wenn beispielsweise die bewegungsarme Phase H₁ sehr kurz ist, während die bewegungsreiche Phase H₂ sehr lang dauert, sind deutlich mehr als zwei Röntgenzyklen erforderlich.

Alternativ kann die Steuerung der Röntgeneinrichtung auch derart erfolgen, dass die einzelnen Röntgenpositionen p₀ bis p₁₆ einzeln nacheinander angesteuert werden, dass in jeder Röntgenposition anhand des Bewegungssignals überprüft wird, ob gerade eine bewegungsarme Phase ermittel ist und dass im positiven Falle ein Projektionsdatensatz ermittelt wird, während im negativen Falle in dieser Röntgenposition gewartet wird, bis wieder eine bewegungsarme Phase erreicht ist, um erst dann den Projektionsdatensatz in dieser Röntgenposition zu erfassen. Auch kann die Ansteuerung der Röntgenpositionen und die Erfassung der Projektionsdaten anhand des Bewegungssignals derart getriggert werden, dass sich die Röntgenquelle immer zu einem festen Zeitpunkt innerhalb einer bestimmten Bewegungsphase in einer neuen Röntgenposition befindet und gleichzeitig ein Korrektionsdatensatz erfaßt wird, so dass alle Projektionsdatensätze zum gleichen Zeitpunkt innerhalb einer Bewegungsphase erfaßt wurden.

In Fig. 5 ist in einem Zeitdiagramm ein Atembewegungssignal B gezeigt, das mit einem in Fig. 1 dargestellten Bauchgurt 9 und einer Atmungsbewegungsmeßvorrichtung 10 ermittelt wurde. Dieses zeigt die Bewegung des Zwerchfells des Patienten 5 in z-Richtung auf. Erkennbar ist, dass die Bewegung des Zwerchfells ebenfalls periodisch erfolgt, dass in ausgeatmetem Zustand B₁ bzw. eingeatmetem Zustand B₃ relativ bewegungsarme Phasen erreicht sind, während dazwischen während des Ein- bzw. Ausatmens relativ bewegungsreiche Phasen B₂ vorliegen. Dieses Atmungsbewegungssignal kann in gleicher Weise benutzt werden wie das anhand der Figuren 3 und 4 erläuterte Herzbewegungssignal H, indem beispielsweise nur während der bewegungsarmen Phasen B₁ Projektionsdatensätze erfaßt werden und die Röntgeneinrichtung entsprechend gesteuert wird. Außerdem kann ein solches Atembewegungssignal auch zusätzlich zu dem Herzbewegungssignal ermittelt werden, um nur solche während bewegungsarmer Phasen H₁ des Herzens ermittelte Projektionsdatensätze auszuwerten, die auch während einer bewegungsarmen Phase B₁, B₃ der Atembewegung ermittelt wurden. Bevorzugt werden dabei auch noch solche Projektionsdatensätze ausgewählt, die während der gleichen Atembewegungsphase, also z.B. alle im ausgeatmetem Zustand, ermittelt wurden, um sich eine zumeist schwierige und zu Ungenauigkeiten führende Korrektur der unterschiedlichen Lage des zu untersuchenden Körperobjektes während eingeatmeter bzw. ausgeatmeter Phase zu vermeiden. Anhand des Atembewegungs- und des Herzbewegungssignals kann die Röntgeneinrichtung auch gezielt derart gesteuert werden, dass einzelne Röntgenpositionen angefahren werden, um dort einen noch fehlenden oder zur falschen Bewegungsphase ermittelten Projektionsdatensatz zu erfassen.

Je nach Anwendung ist es erforderlich, den Patienten kurz vor der Erfassung der Projektionsdatensatze ein Kontrastmittel zu spritzen, um einen ausreichenden Kontrast zwischen dem zu untersuchenden Körperorgan und dessen Umgebung einzustellen. Beispielsweise bei der Untersuchung der Herzkranzgefäße ist dies erforderlich. Da dieses Kontrastmittel jedoch nur eine gewisse Zeit in dem zu untersuchenden Körperorgan verbleibt, beispielsweise ca. 4 Sek. in den Herzkranzgefäßen, aber ein einzelner Röntgenzyklus schon länger dauert, (z.B. 6 bis 9 Sek. bei Röntgenpositionen, die über einen Winkelbereich von 180° um den Patienten verteilt sind), und da ein Kontrastmittel wegen der Belastung für den Patienten nicht beliebig oft gespritzt werden kann, ist es erforderlich, bei solchen Anwendungen das erfindungsgemäße Verfahren derart auszugestalten, dass die Erfassung der erforderlichen Projektionsdatensätze in möglichst kurzer Zeit erfolgen kann. Welche der beschriebenen Ausgestaltungen dabei gewählt wird, hängt jedoch im wesentlichen davon ab, für welche Anwendungen das Verfahren ausgestaltet werden soll, wie viele Projektionsdatensätze für die Ermittlung eines dreidimensionalen Bilddatensatzes bei dieser Anwendung erforderlich sind, wie schnell die Ermittlung eines einzelnen Projektionsdatensatzes erfolgt bzw. wie schnell die einzelnen Röntgenpositionen angefahren werden können und wie groß das Verhältnis der Zeitdauer einer bewegungsarmen Phase zur Zeitdauer einer bewegungsreichen Phase ist.

Die in Fig. 1 gezeigten Mittel zur Messung des Herzensbewegungs- bzw. des Atembewegungssignals sind lediglich beispielhafte Ausgestaltungen. Beispielsweise kann das Herzbewegungssignal auch mittels einer Pulsoxymetrievorrichtung indirekt gemessen werden, während zur Messung des Atembewegungssignals auch eine Ultraschallvorrichtung oder eine Widerstandsmeßvorrichtung zur Messung des elektrischen Widerstandes des Bauchbereichs des Patienten Verwendung finden kann. Auch andere Mittel sind hierzu denkbar. Auch Röntgenröhre und Röntgendetektor können anders ausgestaltet bzw. angeordnet sein. Statt eines Bildverstärkers kann ein flacher zweidimensionaler Digital-Röntgendetektor eingesetzt werden, statt eines C-Bogens mit Röntgenröhre und Röntgendetektor kann grundsätzlich auch eine Computertomographie-Anordnung erfindungsgemäß ausgestaltet werden.

## Patentansprüche

1. Verfahren zur Ermittlung eines dreidimensionalen Bilddatensatzes eines sich periodisch bewegenden Körperorgans (11) eines Patienten (5) mittels einer eine Röntgenquelle (2) und einen Röntgendetektor (3) aufweisenden Röntgeneinrichtung (1), bei dem gleichzeitig zur Ermittlung der Projektionsdatensatze (D₀, D₁, ..., D₁₆) im Zusammenhang mit der periodischen Bewegung des Körperorgans (11) stehendes Bewegungssignal (H, B) ermittelt wird,
dadurch gekennzeichnet,
dass nacheinander die für die Ermittlung des dreidimensionalen Bilddatensatzes erforderlichen Projektionsdatensätze (D₀, D₁, ..., D₁₆) aus unterschiedlichen in einer Ebene liegenden Röntgenpositionen (p₀, p₁, ..., p₁₆) ermittelt werden, dass die Röntgeneinrichtung mittels des Bewegungssignals (H, B) derart gesteuert wird, dass in jeder für die Ermittlung des dreidimensionalen Bilddatensatzes erforderlichen Röntgenposition (p₀, p₁, ..., p₁₆) ein Projektionsdatensatz (D₀, D₁, ..., D₁₆) während einer bewegungsarmen Phase des Körperorgans (11) ermittelt wird, und dass die während der bewegungsarmer Phasen ermittelten Projektionsdatensatze (D₀, D₁, ..., D₁₆) zur Ermittlung des dreidimensionalen Bilddatensatzes verwendet werden.

2. Verfahren nach Anspruch 1,
dadurch gekennzeichnet,
dass zur Ermittlung des dreidimensionalen Bilddatensatzes nur die während gleicher Bewegungsphasen (H₁, B₁) ermittelten Projektionsdatensätze (D₀, D₁, ..., D₁₆) ausgewählt und verwendet werden.

3. Verfahren nach Anspruch 1,
dadurch gekennzeichnet,
dass die verschiedenen Röntgenpositionen (p₀, p₁, ..., p₁₆) in einem Röntgenzyklus (R1) nacheinander durchlaufen werden, dass mehrere Röntgenzyklen (R₁, R₂) aufeinanderfolgend durchlaufen werden und dass die Röntgeneinrichuing (1) mittels des Bewegungssignals (H, B) derart gesteuert wird, dass jeder Röntgenzyklus (R₁, R₂) in einer anderen Bewegungsphase (H₁, H₂; B₁, B₂, B₃) des Körperorgans (11) beginnt.

4. Verfahren nach Anspruch 1,
dadurch gekennzeichnet,
dass die Röntgeneinrichtung (1) mittels des Bewegungssignals (H, B) derart gesteuert wird,
dass Projektionsdatensätze (D₀, D₁, ..., D₁₆) nur während bewegungsarmer Phasen (H₁; B₁, B₃) des Körperorgans (11) ermittelt werden.

5. Verfahren nach Anspruch 1,
dadurch gekennzeichnet,
dass die Röntgeneinrichtung (1) mittels des Bewegungssignals (H, B) derart gesteuert wird,
dass die Röntgenquelle (2) nur während bewegungsarmer Phasen (H₁; B₁, B₃) des Körperorgans (11) zur Ermittlung von Projektionsdatensätzen (D₀, D₁, ..., D₁₆) angeschaltet wird.

6. Verfahren nach Anspruch 1,
dadurch gekennzeichnet,
dass als Bewegungssignal ein von der Atmung des Patienten abhängiges Atembewegungssignal (B) ermittelt wird.

7. Verfahren nach Anspruch 1,
dadurch gekennzeichnet,
dass als Bewegungssignal ein von der Bewegung des Herzens abhängiges Herzbewegungssignal (H), insbesondere ein Elektrokardiogramm, ermittelt wird.

8. Verfahren nach Anspruch 7,
dadurch gekennzeichnet,
dass zusätzlich zum Herzbewegungssignal (H) ein von der Atembewegung abhängiges Atembewegungssignal (B) ermittelt und dass dieses Atembewegungssignal (B) dazu benutzt wird, nur die während gleicher Atembewegungsphasen (B₁) ermittelten Projektionsdatensätze (D₀, D₁, ..., D₁₆) zur Ermittlung des dreidimensionalen Bilddatensatzes zu verwenden.

9. Verfahren nach Anspruch 8,
dadurch gekennzeichnet,
dass das Atembewegungssignal (B) dazu benutzt wird, bei der Ermittlung des dreidimensionalen Bilddatensatzes die in unterschiedlichen Atembewegungsphasen (B₁, B₂, B₃) ermittelten Projektionsdatensätze (D₀, D₁, ..., D₁₆) und sich daraus ergebende Positionsverschiebungen des Körperorgans (11) zu korrigieren.

10. Verfahren nach Anspruch 6, 8 oder 9,
dadurch gekennzeichnet,
dass das Atembewegungssignal (B) dazu benutzt wird, dem Patienten (5) zu signalisieren, wenn eine gewünschte Atembewegungsphase (B₁) erreicht ist, während der die Ermittlung der Projektionsdatensätze (D₀, D₁, ..., D₁₆) erfolgt.

11. Verfahren nach Anspruch 1,
dadurch gekennzeichnet,
dass das Bewegungssignal (H, B) dazu verwendet wird, die Röntgeneinrichrung (1) derart zu steuern, dass Projektionsdatensätze (D₀, D₁, ..., D₁₆) aus einzelnen ausgewählten Röntgenpositionen (p₀, p₁, ..., p₁₆) ermittelt werden.

12. Röntgeneinrichtung, insbesondere zur Durchführung des Verfahrens nach Anspruch 1, mit einer Röntgenquelle (2) und einem Röntgendetektor (3) zur Ermittlung mehrerer Projektionsdatensätze (D₀, D₁, ..., D₁₆) aus unterschiedlichen Röntgenpositionen (p₀, p₁, ..., p₁₆) und zur Ermittlung eines dreidimensionalen Bilddatensatzes eines sich periodisch bewegenden Körperorgans (11) eines Patienten (5) aus den Projektionsdatensätzen (D₀, D₁, ..., D₁₆) und mit Mitteln (7, 8, 9, 10) zur Messung eines im Zusammenhang mit der periodischen Bewegung des Körperorgans (11) stehendes Bewegungssignals (H, B) gleichzeitig zur Ermittlung der Projektionsdatensätze (D₀, D₁, ..., D₁₆),
dadurch gekennzeichnet,
dass eine Rechen- und Steuereinheit (6) vorgesehen ist zur Steuerung der Röntgeneinrichtung (1) und Ermittlung des dreidimensionalen Bilddatensatzes derart, dass nacheinander die für die Ermittlung des dreidimensionalen Bilddatensatzes erforderlichen Projektionsdatensatze (D₀, D₁, ..., D₁₆) aus unterschiedlichen in einer Ebene liegenden Röntgenpositionen (p₀, p₁, ..., p₁₆) ermittelt werden, dass in jeder für die Ermittlung des dreidimensionalen Bilddatensatzes erforderlichen Röntgenposition (p₀, p₁, ..., p₁₆) ein Projektionsdatensatz (D₀, D₁, ..., D₁₆) während einer bewegungsarmen Phase des Körperorgans (11) ermittelt wird und dass nur die während bewegungsarmer Phasen ermittelten Projektionsdatensätze (D₀, D₁, ..., D₁₆) zur Ermittlung des dreidimensionalen Bilddatensatzes verwendet werden.

13. Röntgeneinrichtung nach Anspruch 12,
dadurch gekennzeichnet,
dass die Mittel (7, 8) zur Messung des Bewegungssignals ausgestaltet sind zur Messung eines von der Herzbewegung abhängigen Herzbewegungssignals (H).

14. Röntgeneinrichtung nach Anspruch 12,
dadurch gekennzeichnet,
dass die Mittel (7, 8) zur Messung des Herzbewegungssignals (H) eine Elektrokardiographieeinrichtung oder eine Pulsoxymetrievorrichtung aufweisen.

15. Röntgeneinrichtung nach Anspruch 12,
dadurch gekennzeichnet,
dass die Mittel (9, 10) zur Messung des Bewegungssignals ausgestaltet sind zur Messung eines von der Atembewegung abhängigen Atembewegungssignals (B).

16. Röntgeneinrichtung nach Anspruch 15,
dadurch gekennzeichnet,
dass eine Signaleinrichtung (12) vorgesehen ist zur Signalisierung des Erreichens einer gewünschten Atembewegungsphase (B₁) an den Patienten (5).

17. Röntgeneinrichtung nach Anspruch 15,
dadurch gekennzeichnet,
dass die Mittel (9, 10) zur Messung des Atembewegungssignals (B) eine Ultraschallvorrichtung, einen Bauchgurt zur Messung der Bewegung des Zwerchfells oder eine Widerstandsmeßvorrichtung zur Messung des Widerstands des Bauchbereichs des Patienten (5) aufweisen.
